(19) Europäisches Patentamt
European Patent Office
Office européen des brevets

(11) **EP 4 741 004 A1**

(12) **EUROPEAN PATENT APPLICATION**

(43) Date of publication:
**13.05.2026 Bulletin 2026/20**

(21) Application number: **24212019.4**

(22) Date of filing: **11.11.2024**

(51) International Patent Classification (IPC):
**A61N 5/10** $^{(2006.01)}$

(52) Cooperative Patent Classification (CPC):
**A61N 5/1038;** A61N 5/1031; A61N 2005/1041

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Siemens Healthineers International AG**
**6312 Steinhausen (CH)**

(72) Inventors:
• **KUUSELA, Esa**
  **02320 Espoo (FI)**
• **PELTOLA, Jarkko**
  **04300 Tuusula (FI)**

(74) Representative: **Mathisen & Macara LLP**
**Charta House**
**30-38 Church Street**
**Staines-upon-Thames TW18 4EP (GB)**

(54) **UTILITY FUNCTIONS IN RADIATION TREATMENT PLANNING**

(57)    A method (300) for computer-assisted radiation therapy treatment planning, comprising: receiving (310) a desired baseline dose distribution based on dose distributions used in radiation therapy treatment planning for one or more past patients that are similar to a new patient; receiving or defining (320) a set of clinical goals; using the baseline dose distribution to calibrate (340) a utility function or cost function based on the set of clinical goals; and using the utility function or cost function to optimise (350) a treatment plan.

300

310 — Receive desired baseline dose distribution

320 — Receive / define a prioritised list of clinical goals

330 — Define significant / insignificant deviations from clinical goals

340 — Calibrate cost or utility function using baseline dose distribution, clinical goals, clinical goal priorities, and information regarding significant / insignificant deviations from clinical goals

350 — Optimise planned dose distribution and planned machine parameters by minimising cost function / maximising utility function

360 — Obtain treatment plan

FIG. 3

EP 4 741 004 A1

## Description

### TECHNCAL FIELD

This invention relates to radiation therapy treatment planning, and in particular, to methods and systems for construction of a utility or cost function usable to develop a radiation therapy treatment plan.

### BACKGROUND

The use of energy to treat medical conditions comprises a known area of prior art endeavour. For example, radiation therapy comprises an important component of many treatment plans for reducing or eliminating unwanted tumours. Unfortunately, applied energy does not inherently discriminate between unwanted material and adjacent tissues, organs, or the like that are desired or even critical to continued survival of the patient (such adjacent tissues may be referred to as "organs at risk", OARs). As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform "machine" parameters during each of a plurality of sequential fields. For example, machine parameters of a radiotherapy system may include the angle of irradiation of the treatment beam, or the configuration of the leaves of a multileaf collimator.

Treatment plans for radiation treatment sessions are often automatically generated through a so-called optimisation process. As used herein, "optimisation" will be understood to refer to improving a candidate treatment plan without necessarily ensuring that the optimised result is, in fact, the singular best solution. Such optimisation often includes automatically adjusting one or more physical treatment machine parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

In some cases, optimisation proceeds as a function of multiple different criteria. This may involve the use of a cost function comprising a mathematical model that attempts to balance conflicting clinical goals to produce a treatment plan with the least "cost". Clinical goals generally take the form of a particular metric being required to be greater or smaller than a threshold value. For example, a clinical goal may require that a certain percentage of the volume of an OAR receives less than a certain amount of dose. As a further example, a clinical goal may require that a certain percentage of the planning target volume (PTV) receives at least a certain amount of dose. A cost function may be a sum of terms relating to deviations from the clinical goals.

As an alternative to a cost function, a utility function may be used to attempt to balance the clinical goals. A utility function is similar to a cost function, except that the optimisation attempts to produce a treatment plan with the highest "utility", rather than the lowest "cost". For the rest of this disclosure, the maximisation of a utility function or the minimisation of a cost function reflect complementary mathematical methods that can be used in similar ways.

A computer-implemented optimiser is generally used to iteratively maximise (or minimise) the utility function (or cost function). In general, the process of optimisation involves finding a set of machine parameters (also referred to as "control points") that maximise a given utility function (or minimise the cost function).

Traditionally, a utility function or cost function is developed on the basis of a desired dose distribution. However, the choice of a dose distribution that is used as a starting point for the optimisation process is usually based on an idealised dose to each of the regions of interest, which may be difficult to achieve. Further, while the concept of prioritisation of clinical goals during the optimisation process is known, conventional methods do not take into account what may be clinically insignificant deviations from a goal, or what may be clinically significant deviations from the goal.

### SUMMARY OF THE INVENTION

According to a first aspect of the invention, there is provided a method for computer-assisted radiation therapy treatment planning as defined by claim 1.

According to a second aspect of the invention, there is provided a radiation treatment planning computer system as defined by claim 14.

According to a third aspect of the invention, there is provided a computer program product as defined by claim 15.

Optional features are defined by the dependent claims.

According to the first aspect of the invention, there is provided a method for computer-assisted radiation therapy treatment planning, comprising: receiving a desired baseline dose distribution based on dose distributions used in radiation therapy treatment planning for one or more past patients that are similar to a new patient; receiving or defining a set of clinical goals; using the baseline dose distribution to calibrate a utility function or cost function based on the set of clinical goals; using the utility function or cost function to optimise a treatment plan.

The received desired baseline dose distribution may be based on a dose distribution used in previous radiation therapy treatment planning for the same (i.e., the new) patient. For example, the baseline dose distribution for the new patient may comprise a pre-optimised / planned dose distribution that was previously determined for the new patient. So, when starting planning with a new patient, the cost/utility functions may be calibrated based on a previous plan for the new patient, e.g. transferred from another system.

The baseline dose distribution may be a 3D dose matrix.

In some prior art examples, machine learning models can be trained using a cohort of similar plans for different patients, and when applied to a new patient, an estimate of a dose volume histogram (DVH) may be made based on the past knowledge. These DVH estimations may be used to determine goal metrics and optimisation objectives. In contrast, in some embodiments a comprehensive baseline dose distribution comprising a 3D dose matrix is provided that can be used to determine any arbitrary suitable metric for calibration of a utility function or a cost function.

In some embodiments, the received desired baseline dose distribution comprises a dose distribution used for a past patient similar to the new patient.

Advantageously, by using a baseline dose distribution that is similar to a dose distribution of a similar past patient, an optimisation starting point that is closer to an achievable dose distribution is obtained.

In some embodiments, the method includes selecting a dose prediction model constructed for past patients similar to the new patient, from a library of dose prediction models; and using the selected dose prediction model to generate the desired baseline dose distribution.

In some embodiments, the method includes selecting a template utility function or a template cost function constructed for past patients similar to the new patient, from a library of template utility functions or template cost functions; and using the selected template utility function or selected template cost function to generate the desired baseline dose distribution.

In some embodiments, the received desired baseline dose distribution is partially or wholly defined by a human user.

In some embodiments, the received desired baseline dose distribution is further modified by a human user.

In some embodiments, the received desired baseline dose distribution is partially or wholly defined by a computer-implemented method.

In some embodiments, the baseline dose distribution is generated based on relaxed machine parameter constraints.

In some embodiments, the method further comprises receiving information defining one or more of: a relative priority of each clinical goal; significant deviations from clinical goals, and/or insignificant deviations from clinical goals; and using the baseline dose distribution and the received information defining the relative priority of each clinical goal, significant deviations from clinical goals, and/or insignificant deviations from clinical goals to calibrate the utility function or cost function.

In some embodiments, the information defining clinical goal priorities, significant deviations from clinical goals and/or insignificant deviations from clinical goals is received from a library of templates defining clinical goal priorities, significant deviations from clinical goals and/or insignificant deviations from clinical goals in past similar patients.

In some embodiments, the information defining clinical goal priorities, significant deviations from clinical goals and/or insignificant deviations from clinical goals is at least partially received from a human user.

A lower priority goal may take precedence over a higher priority goal, in dependence on, during optimisation, the significance of a change to a lower priority metric and/or the significance of a change to a higher priority metric. For example, an optimisation iteration that results in a significant improvement of a lower priority metric and an insignificant worsening of a higher priority metric may be accepted. Additionally or alternatively, an optimisation iteration that results in an insignificant improvement of a higher priority metric but a significant worsening of a lower priority metric may not be accepted.

In some embodiments, the cost function comprises a sum of quadratic terms, each quadratic term penalising a deviation from a corresponding clinical goal.

In some embodiments, each quadratic term is normalised by significance.

In some embodiments, the utility function or cost function comprises a piecewise-linear function.

In some embodiments, the utility function or cost function is assessed by determining a rating that depends on the extent to which the desired baseline dose distribution is achieved.

In some embodiments, the rating is a maximal value when all clinical goals are achieved; the rating is a minimal value when one or more of achieved metrics deviate from corresponding clinical goals by more than a significant amount; and the rating is between the maximal value and the minimal value in all other cases.

In accordance with the second aspect of the invention, there is provided a radiation treatment planning computer system comprising a memory and a processor configured to perform the method as defined above.

In particular, in accordance with the second aspect of the invention, there is provided a radiation treatment planning computer system comprising a memory and a processor configured to perform a method for computer-assisted radiation therapy treatment planning, the method comprising: receiving a desired baseline dose distribution based on dose distributions used in radiation therapy treatment planning for one or more past patients that are similar to a new patient; receiving or defining a set of clinical goals; using the baseline dose distribution to calibrate a utility function or cost function based on the set of clinical goals; and using the utility function or cost function to optimise a treatment plan.

In accordance with the third aspect of the invention, there is provided a computer program product comprising a non-transitory, computer readable storage medium encoded with instructions operable for execution by a processor to perform the above method.

In particular, in accordance with the third aspect of the invention, there is provided a computer program product comprising a non-transitory, computer readable storage medium encoded with instructions operable for execution by a processor to perform the method comprising: receiving a desired baseline dose distribution based on dose distributions used in radiation therapy treatment planning for one or more past patients that are similar to a new patient; receiving or defining a set of clinical goals; using the baseline dose distribution to calibrate a utility function or cost function based on the set of clinical goals; and using the utility function or cost function to optimise a treatment plan.

In accordance with a fourth aspect of the invention, there is provided a radiation treatment planning computer system comprising:

means for receiving a desired baseline dose distribution based on dose distributions used in radiation therapy treatment planning for one or more past patients that are similar to a new patient;
means for receiving or defining a set of clinical goals;
means for using the baseline dose distribution to calibrate a utility function or cost function based on the set of clinical goals; and
means for using the utility function or cost function to optimise a treatment plan.

By using a "baseline dose distribution" in the definition of a utility function or cost function, radiation treatment plans may be obtained more efficiently. Further, by using significance of deviation from clinical goals in the definition of the utility / cost function, superior radiation treatment plans may be obtained.

## BRIEF DESCRIPTION OF THE DRAWINGS

For a better understanding of the present disclosure, embodiments will now be described by way of example with reference to the accompanying drawings, in which:

FIG. 1       is an illustration of a typical radiotherapy treatment system;
FIG. 2       is a side view of a radiotherapy treatment system in use;
FIG. 3       is a flowchart of a method to optimise a radiotherapy treatment plan using a baseline dose distribution and information defining significant and/or insignificant deviations from clinical goals;
FIG. 4       is a table showing acceptance or rejection of optimisation iteration results on the basis of whether improvements or worsening of a high priority goal metric or a low priority goal metric have taken place;
FIG. 5       is a flowchart of a method to obtain a baseline dose distribution using a dose prediction model in a knowledge base of past patients;
FIG. 6       is a flowchart of a method to obtain a baseline dose distribution using a past utility function or a past cost function used in similar past patients;
FIG. 7A       shows an example of a quadratic cost function;
FIG. 7B       shows an example of a piecewise linear cost function;
FIG. 7C       shows an example of a smooth cost function; and
FIG. 8       shows a typical computer system upon which the present method may be implemented.

## DETAILED DESCRIPTION

Treatment system

In general, radiation therapy consists of the use of ionising radiation to treat living tissue, usually tumours. There are many different types of ionising radiation used in radiation therapy, including high energy x-rays, electron beams, and proton beams. The process of administering the radiation to a patient can be somewhat generalised regardless of the type of radiation used. External beam therapy (EBT), also called external radiation therapy, is a method for delivering a beam or several beams of high-energy x-rays to a patient's tumour. Beams are generated outside the patient (usually by a linear accelerator) and are targeted at the tumour site.

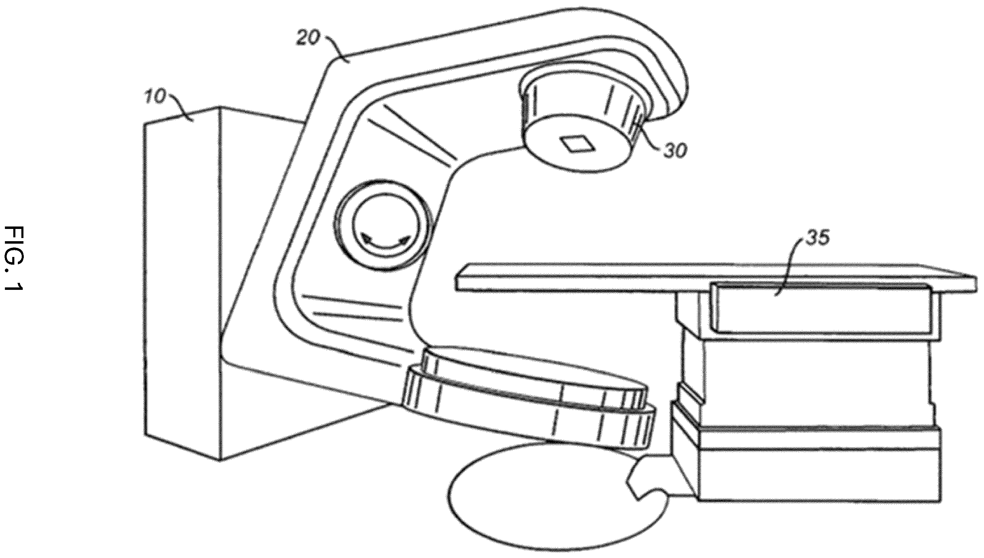

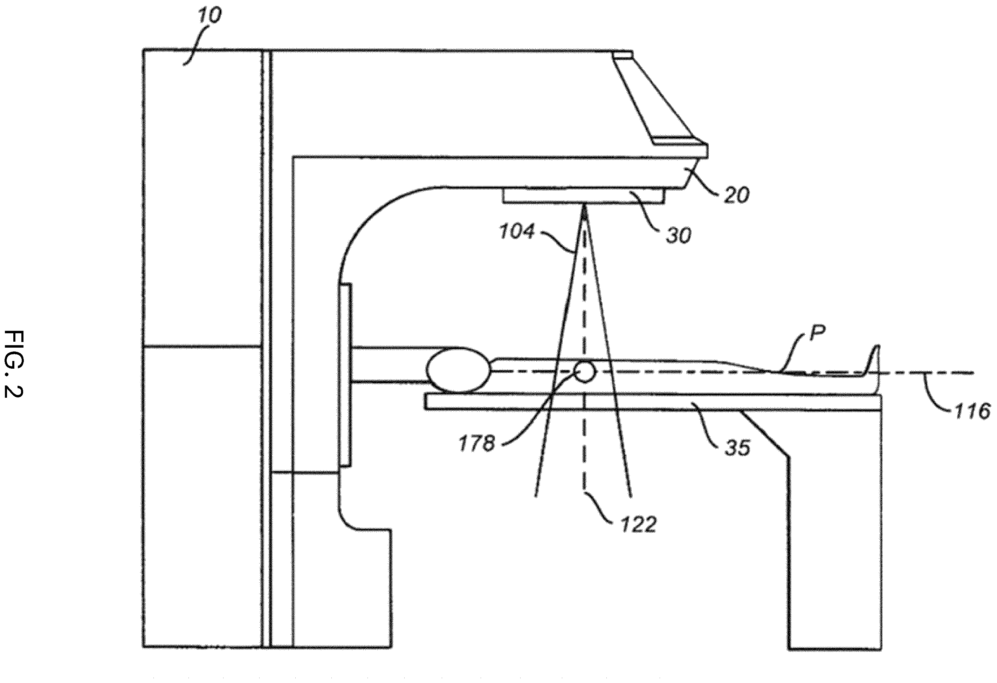

FIGS. 1 and 2 depict a radiation treatment system of the type that may be used in accordance with some embodiments. Referring to FIG. 1, a perspective view of a radiation treatment system (in this case a linear accelerator) is shown. Typically, such a system is capable of generating either an electron (particle) beam or an x-ray (photon) beam for use in the radiotherapy treatment of patients on a treatment couch 35. Other radiation treatment systems are capable of generating heavy ion particles such as protons. For purposes of the present discussion, only x-ray irradiation will be discussed. However, it will be appreciated by those skilled in the art that the same principles apply to other (e.g. particle beam) systems.

Stand 10 supports a rotatable gantry 20 with a treatment head 30. Next to stand 10 there is arranged a control unit (not shown) that includes control circuitry for controlling the different modes of operation of the accelerator. A high voltage source is provided within the stand or in the gantry, to supply voltage to an electron gun (not shown) positioned on an accelerator guide located in the gantry 20. Electrons are emitted from the electron gun into the guide (not shown) where they are accelerated. A source supplies RF (microwave) power for the generation of an electric field within the waveguide. The electrons emitted from the electron gun are accelerated in the waveguide by the electric field, and exit the waveguide as a high energy electron beam, typically at megavoltage energies. The electron beam then strikes a suitable metal target, emitting high energy x-rays in the forward direction.

Referring now to FIG. 2, a somewhat more detailed side view of a radiation treatment system is shown. A patient P is shown lying on the treatment couch 35. X-rays formed as described above are emitted from the target in the treatment head 30 in a divergent beam 104. Typically, a patient plane 116, which is perpendicular to the page in FIG. 2, is positioned about one meter from the x-ray source or target, and the axis of the gantry 20 is located on the plane 116, such that the distance between the target and the isocentre 178 remains constant when the gantry 20 is rotated. The isocentre 178 is at the intersection between the patient plane 116 and the central axis of beam 122. A treatment volume to be irradiated is located about the isocentre 178.

Although the above discussion relates to external beam therapy, it should be understood that the present invention is not so limited, and may, for example, be used with other types of radiation therapy such as brachytherapy.

Plan optimisation using baseline and significance

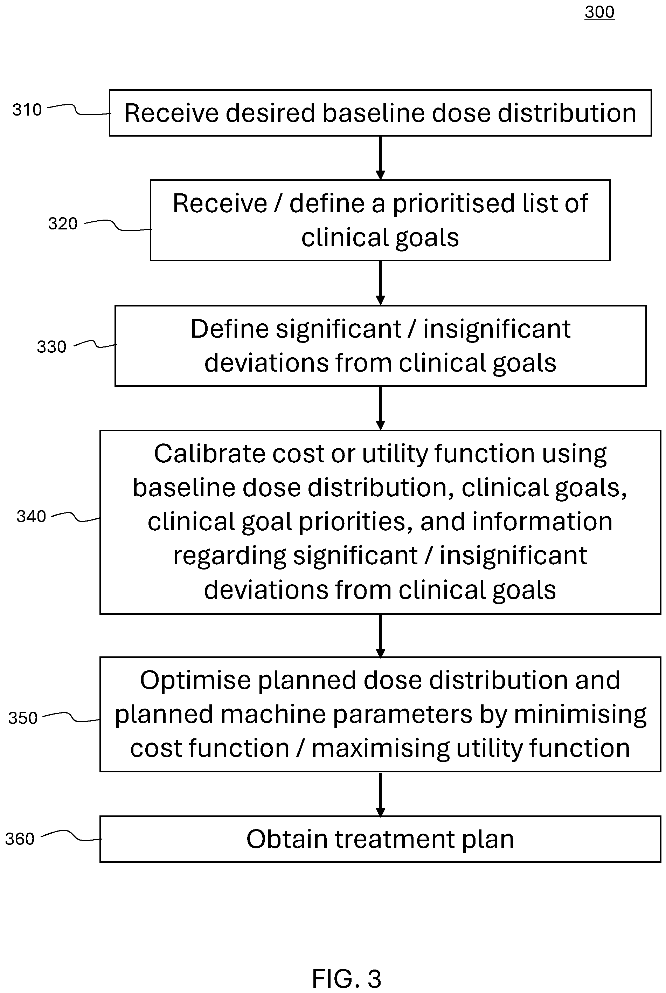

FIG. 3 is a flowchart of a radiation therapy treatment plan optimisation method in accordance with some embodiments.

*Baseline dose distribution*

In step 310, the radiation therapy treatment plan development process begins by determining or receiving a desired baseline dose distribution. A "baseline" refers to a dose distribution that is used as a starting point for an optimisation process. For example, rather than starting from a fully "ideal" dose distribution for a particular patient, dose distributions achieved for similar past patients may be used as a starting point instead. Such a distribution used as a starting point in the optimisation process is referred to as a baseline dose distribution.

A baseline dose distribution may also be defined as a "reference" dose which is not an ideal dose but is closer to what is achievable in a real system than an ideal dose. Thus, instead of the plan optimiser attempting to produce a prior art dose distribution that is too difficult to achieve, the plan optimiser only needs to achieve a baseline dose distribution, wherein the baseline dose distribution may provide an easier starting point for the optimiser. Thus, the "reference" dose re-defines the highest plan quality value required to be produced by a utility function or cost function, such that the required dose is re-defined from an ideal dose distribution to a baseline dose distribution.

The baseline dose distribution may be closer to a realistic or achievable dose distribution, although the baseline dose distribution does not have to be actually realisable. The closer the baseline dose distribution is to a realistic dose distribution, the more accurately the planner can describe the optimisation problem relative to what is achievable. If an ideal dose distribution is used as a starting point for the optimisation without taking into account what can actually be delivered, then the final optimised treatment plan will differ greatly from the starting dose distribution. By starting from a baseline dose distribution, the final optimised treatment plan can produce a dose distribution that is closer to the starting dose distribution.

The definition of a relatively "realistic" or "close to achievable" dose would differ from patient to patient, and would depend on patient geometry. For example, a "realistic" dose may depend on details of the patient's anatomy, the defined target structures and organs at risk, and/or the prescription levels. Thus, the same set of clinical goals used for two different patients may result in a dose distribution that is too difficult to achieve for one patient, but that is "too easy" to achieve for another patient. In the case where the clinical goals are "too easy", the bar is set too low, and the optimiser could obtain a superior treatment plan by making the clinical goals more conformal to the patient anatomy but harder to achieve. Thus, in some embodiments, a realistic baseline dose distribution is selected that is suitable for and tailored for each individual patient.

Using a baseline dose distribution as a starting point for an optimisation process, superior clinical goals are defined that are appropriately ambitious yet that are also achievable and patient-specific.

Although the optimisation process should, when complete, produce a treatment plan that is realisable by a radiation therapy treatment system, as noted above the baseline dose distribution does not have to be realisable by such a system. That is, the baseline dose distribution is "relaxed" with respect to machine parameter constraints. For example, given the available degrees of freedom of a multileaf collimator and a gantry, it may be physically impossible to achieve the baseline dose distribution. This is because the baseline dose distribution is used to provide a better starting point for the optimisation process, and does not comprise the final output of the optimisation process, which would need to be realisable by the treatment system.

By choosing an appropriate baseline dose distribution, a realistic achievable treatment plan, that differs less from the baseline dose distribution than from an ideal dose distribution, may be obtained.

As a further point, by starting from a baseline dose distribution rather than an ideal dose distribution, the optimisation process arrives at a final solution faster and more efficiently.

As will be described in detail below, the baseline dose distribution is used to calibrate a utility function or a cost function to be used in an optimisation process. For example, in addition to including prior-art idealised clinical goals in a utility function or a cost function, in some embodiments clinical goals based on a baseline dose distribution may be used in the cost function or utility function.

A baseline dose distribution may be defined (i) wholly by a computer-implemented process, (ii) wholly by user input, or (iii) partially by user input and partially by a computer-implemented process.

In some embodiments, the meaning of "dose" in the term "baseline dose distribution" may include monitor unit count.

*Priority and significance*

Returning to FIG. 3, in step 320, a prioritised list of clinical goals is defined. The prioritised list of clinical goals may be obtained from such lists used in similar past patients. For example, the user may have access to an initial clinical goal template for similar past patients, where the template is a prioritised list of clinical goals.

In step 330, information defining significant deviations from clinical goals may be defined. Alternatively, information defining insignificant deviations from clinical goals may be defined. In some embodiments, information defining both significant and insignificant deviations from clinical goals may be defined.

Information defining clinical goal priorities, significant deviations from clinical goals and/or insignificant deviations from clinical goals may be received from a library of templates defining clinical goal priorities, significant deviations from clinical goals and/or insignificant deviations from clinical goals in past similar patients. In some embodiments, information defining clinical goal priorities, significant deviations from clinical goals and/or insignificant deviations from clinical goals may at least be partially received from a human user. In some embodiments, information defining clinical goal priorities, significant deviations from clinical goals and/or insignificant deviations from clinical goals may be obtained by a computer-implemented optimisation process that determines this information based on how to achieve the baseline dose distribution.

That is, information on significant or insignificant deviations can be automatically generated from known default values used for past patients. For example, this may include information on clinically known and significant deviations such as deviations from the maximum dose to the spine.

*Using significance*

As noted above, in some embodiments, the clinical goals are listed in order of priority. In general, in the prior art, the optimiser attempts to meet as many of the clinical goals as possible, except that it would be more important to meet a higher priority goal than it would be to meet a lower priority goal. In such arrangements, any iteration of the optimisation process that results in more closely meeting a low-priority goal at the expense of deviating from a high-priority goal, would be rejected. An exemplary list of prioritised clinical goals is provided in Table 1 below.

Table 1: Example of prioritised list of clinical goals

| Lt Parotid | (P1.1) D50.0 % ≤ 20.00 Gy | ☑ 19.15 Gy |
|---|---|---|
| Rt Parotid | (P1.2) D50.0 % ≤ 20.00 Gy | ☑ 19.46 Gy |
| PTV | (P2.1) D90.0 % ≥ 50.00 Gy | ☑ 51.43 Gy |

(continued)

| | | |
|---|---|---|
| **PTV** | (P2.2) D99.0 % $\geq$ 46.50 Gy | ☑ 47.00 Gy |
| **PTV** | (P2.3) D20.0 % < 55.00 Gy | ☑ 54.63 Gy |
| **Cord** | (P2.4) Dmax(0.0%) < 40.00 Gy | ☑ 32.86 Gy |
| **BODY** | *Dmean$\leq$ 50.00 Gy* | ☑ 8.38 Gy |
| **BODY** | *Dmax(0.0%) $\leq$ 50.00 Gy* | ☒ 56.50 Gy |
| **PTV** | *Dmean$\leq$ 50.00 Gy* | ☒ 53.29 Gy |
| **PTV** | *Dmax(0.0 %) $\leq$ 50.00 Gy* | ☒ 56.50 Gy |

In the above table, "Lt Parotid" and "Rt Parotid" refer to the left and right parotid glands, respectively. The "PTV" refers to the planning target volume. The "Cord" refers to the spinal cord. The "BODY" refers to the whole patient volume that is imaged. D50% < x means that 50% of the respective organ should receive less than x dose. The table above has other similar goals with a similar meaning to D50% < x (e.g., D90% > x has a similar meaning). Dmax refers to the maximum delivered dose. Dmean refers to the mean delivered dose. Clinical goals are more fully described below.

The clinical goals are arranged in groups. For example, in the above table, P1.2 means that the clinical goal is the second most important goal in group 1. The right-most column for each goal is what was achieved from the optimisation of a plan for this exemplary patient. The tick indicates a clinical goal that has been achieved. The cross indicates a clinical goal that has not been achieved.

In some embodiments, in addition to listing the clinical goals in order of priority, significant and insignificant deviations from the clinical goals are also defined. In such embodiments, under certain conditions a lower priority goal may take precedence over a higher priority goal. For example, an optimisation iteration that results in a significant improvement of a lower priority metric is accepted if there is only an insignificant worsening of a higher priority metric. Similarly, although an improvement of a higher priority metric at the expense of a lower priority metric would normally be accepted, if this comprises only an insignificant improvement of the higher priority metric but a significant deterioration of the lower priority metric, this may not be accepted.

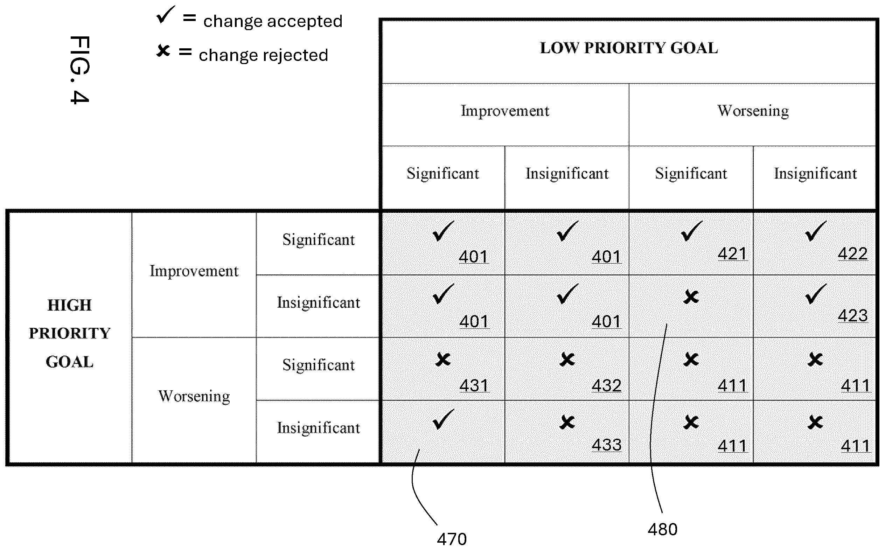

FIG. 4 is a table showing whether significant or insignificant improvements or worsening of high priority and low priority goal metrics are accepted or rejected. Improvements in both a high priority metric and a low priority metric are always accepted, as shown by 401. Worsening of both a high priority metric and a low priority metric are always rejected, as shown by 411. On the other hand, an improvement of a low priority metric at the expense of a worsening of high priority metric, which would normally be rejected, is accepted if the low priority metric improves significantly and the high priority metric deteriorates only insignificantly, as shown by 470. Further, an improvement of a high priority metric at the expense of a worsening of a low priority metric, which would normally be accepted, is rejected if the high priority metric only improves insignificantly, and the low priority metric worsens significantly, as shown by 480.

A significant improvement in a high priority metric with a significant or insignificant worsening of a low priority metric is generally accepted, as shown by 421, 422. However, if the high priority metric significantly improves at the expense of a significant worsening of a low priority metric, where the degree of significance of worsening of the low priority metric is greater than the degree of significance of an improvement in the high priority metric, this may be rejected.

An insignificant improvement in a high priority metric with an insignificant worsening in a low priority metric is generally accepted, as shown by 423.

A significant worsening of a high priority metric with a significant or insignificant improvement of a low priority metric is generally rejected, as shown by 431, 432. However, if the degree of significance of improvement of the low priority metric is greater than the degree of significance of worsening of the high priority metric, this may be accepted.

An insignificant improvement of a low priority metric with an insignificant worsening of a high priority metric is generally rejected, as shown by 433.

In some embodiments, cost functions and/or utility functions may be developed that include terms related to the significance of improvements or worsening with respect to one or more clinical goals. For example, cost functions and/or utility functions may comprise terms related to the significance of deviations from one or more clinical goals.

Advantageously, by specifying significant or insignificant improvements or worsening of high priority and low priority metrics, the optimisation provides a radiation treatment plan that is more suitable for meeting the individual clinical goals of each patient, and better suited to the particular tumour and/or OAR geometry of the patient.

*Examples of using priority and significance*

With reference to the table of exemplary clinical goals provided above (i.e., Table 1), in one scenario, the spinal cord may be relatively close to the PTV. In this case, it may be that in order to meet a PTV coverage goal of D99.0% >= 46.50 Gy a hotspot of 42 Gy is required to be delivered to the spine ("Cord"). This may lead to a violation wherein for the spine, the clinical goal is Dmax < 40.00 Gy. Thus, a higher priority metric is improved at the expense of a lower priority metric. If this violation of the goal dose to the spine is defined as insignificant, the violation would be considered acceptable.

However, in an example where the PTV coverage D99.0% is actually 46.40 Gy and the spinal cord Dmax is 37.0, there is a worsening of a higher priority metric with an improvement of a lower priority metric. In prior art solutions, this would typically be considered unacceptable. However, in this example there is an insignificant worsening of a higher priority metric but a significant improvement of a lower priority metric. In accordance with some embodiments, since with a very small absolute change in the PTV coverage, a significant improvement in the spinal cord Dmax is obtained, this change in the metric values is considered to be acceptable.

As a further example, a clinical goal may require that the maximum dose to the spine must be 40Gy. However, this would only be the barely acceptable treatment limit. It may be desirable to achieve a lower dose to the spine. Here, in accordance with some embodiments, the significance of achieving lower doses to the spine may be defined. For example, it may be defined that achieving a dose of 20Gy to the spine would be a significant improvement of the treatment plan. However, achieving an even lower dose to the spine, such as 10Gy, may be defined as being an insignificant improvement of the treatment plan. That is, the use of significance in construction of the utility function or cost function defines how significant improvements or deterioration of the treatment plan is with respect to the prioritised clinical goals.

In some embodiments, information on significance may be considered as part of the baseline dose distribution.

*Calibration & optimisation*

Returning to FIG. 3, in step 340, a cost function or utility function may be calibrated using the received baseline dose distribution, the clinical goals, the clinical goal priorities, and the information regarding significant and/or insignificant deviations from the clinical goals. For example, the terms of a quadratic cost function that may define a weighted sum of the squared deviations from clinical goals of a radiation therapy treatment plan, may be calibrated or adjusted using the baseline dose distribution, the information regarding the clinical goal priorities and the information regarding significant and/or insignificant deviations from clinical goals.

By "fine-tuning" or calibrating a cost function or utility function to a baseline dose distribution rather than an ideal dose distribution, and by using cost or utility terms that take into account significance of deviations from clinical goals, the cost function or utility function is able to drive the optimisation process to achieve a result closer to a more realistic dose distribution and that is closer to being achievable (yet more conformal to the patient anatomy) than is possible using prior art solutions.

For example, the optimisation using the significance information in the cost functions or utility functions according to some embodiments may result in a low priority goal taking precedence over a high priority goal if a significant improvement is achieved for the low priority goal, but only an insignificant deterioration of the high priority goal is obtained. Similarly, the use of significance in the cost functions or utility functions may result in a low priority goal taking precedence over a high priority goal if an insignificant improvement in a high priority goal results in a significant deterioration of a low priority goal.

Prior art systems do not use the notion of significance to calibrate or adjust a cost function or a utility function, and by using significance in a cost function or utility function in accordance with some embodiments, superior treatment plans are developed which are more suitable for the individual anatomy of a particular patient.

In step 350, the cost function or utility function calibrated in step 340 is used to optimise a radiation therapy treatment plan and provide a set of machine parameters, which when implemented in a radiation therapy treatment system, provides a dose distribution that is as close as possible to the baseline dose distribution, taking into account prioritised clinical goals and significance information. In particular, any optimisation scheme may be used to obtain a set of machine parameters that minimise a cost function or maximise a utility function determined using a baseline dose distribution, prioritised clinical goals and significance. Such optimisation schemes may include, for example, simulated annealing, column generation, and/or differential evolution. A skilled person would be aware of how each of these optimisation schemes is used, and so these require no elaboration here.

In some embodiments, a single optimisation is performed with a calibrated cost function or utility function. This may be referred to as "single-shot" optimisation. In a single-shot optimisation, multiple iterations are run using the same utility function or cost function. In some embodiments, multiple optimisations are performed with a modified cost function or utility function used during each optimisation. For each of the multiple optimisations, the modified cost function or utility function may be provided by a human user or provided by a computer-implemented method using information from past patient treatments.

By using a baseline dose distribution and significance information in the optimisation process, the number of optimisations or modifications of the cost function or utility function is reduced and fewer "trials" are required to reach an acceptable plan. In particular, by using a baseline dose distribution and significance information to develop the utility function or cost function, it is easier, or made possible, to perform the optimisation as a "single-shot" optimisation. However, this would depend on how realistic and/or how idealistic the baseline dose distribution is. A relatively idealistic baseline dose distribution may require modifications of the utility function or cost function over a number of optimisations, whereas a relatively realistic baseline dose distribution may require fewer or only one round of optimisation.

In step 360, a treatment plan is obtained as the output or result of the radiation therapy treatment optimisation process 350. In contrast to prior art schemes, the present scheme uses significance information and a baseline dose distribution in the optimisation process, as defined above.

## Obtaining the baseline dose distribution

A baseline dose distribution may be obtained by human user input, by a computer-implemented method, or by both human user input and a computer-implemented method. In some embodiments, a computer-implemented method to obtain a baseline dose distribution may use a dose prediction model selected from a knowledge base of similar past patients. In some embodiments, a computer-implemented method to obtain a baseline dose distribution may use a past utility function or past cost function obtained from a library of utility function templates or cost function templates.

### *Knowledge base of dose prediction models*

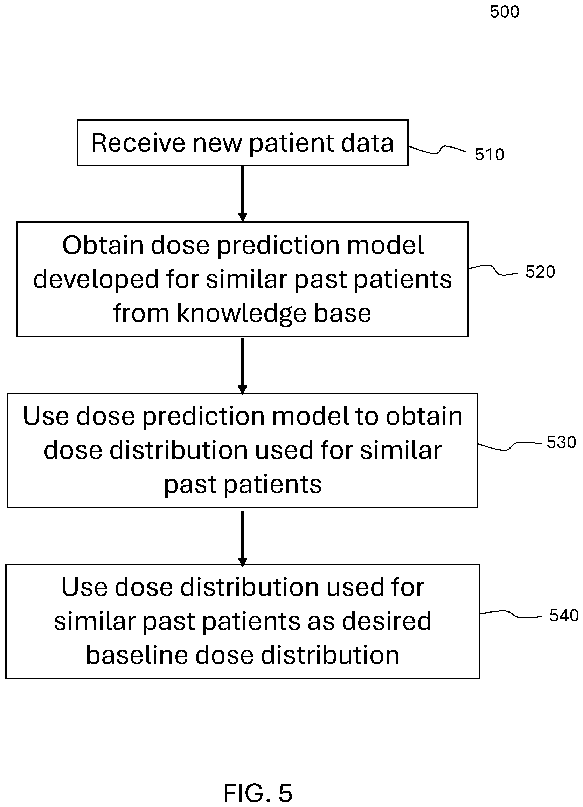

FIG. 5 is a flowchart of obtaining a baseline dose distribution using a dose prediction model selected from a knowledge base of past patients.

In step 510, new patient data is received.

In step 520, a dose prediction model developed for similar past patients is obtained from a knowledge base. A knowledge base may comprise a database containing past treatment history of past patients.

In step 530, the dose prediction model is used to obtain a dose distribution that was used for similar past patients.

As an optional further step, a human user may make some modifications to the dose distribution obtained from the dose prediction model. For example, an expert highly experienced treatment planner may notice that a certain metric associated with the dose distribution obtained from the dose prediction model is not acceptable, and may make amendments to the dose distribution accordingly.

In step 540, the dose distribution obtained from the obtained dose prediction model (and possibly modified by the human user) is used as a baseline dose distribution. For example, a baseline dose distribution obtained in step 540 may be used in the method as illustrated in FIG. 3 and as described above in relation to FIG. 3; that is, the baseline dose distribution obtained in step 540 may be used as a starting point in a radiation therapy treatment plan optimisation process.

### *Library of utility functions*

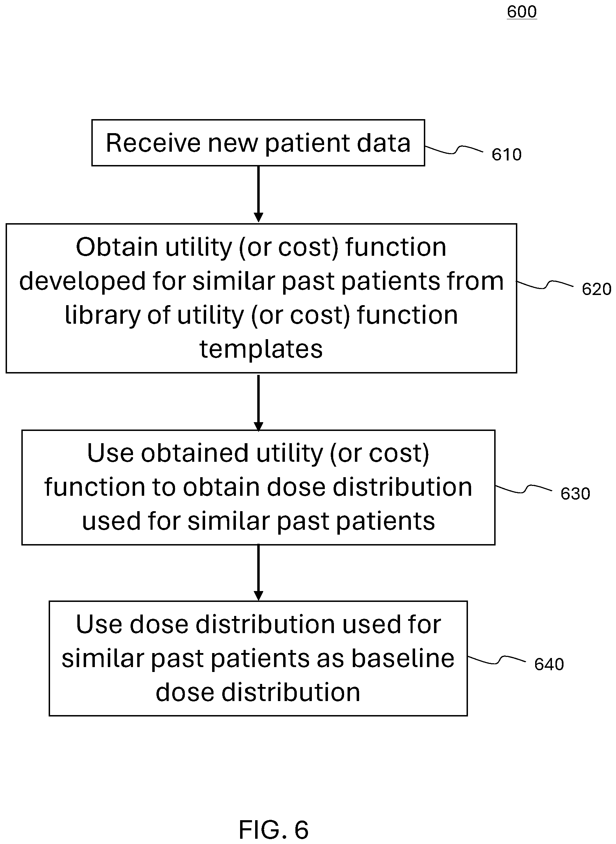

FIG. 6 is a flowchart of obtaining a baseline dose distribution using a past utility function or past cost function developed for similar past patients.

In step 610, new patient data is received.

In step 620, a utility function or cost function that was developed for similar past patients is obtained from a library of utility function templates and/or cost function templates.

The library may have different templates for different patient geometry groups. For example, liver-treatment patients might have one common template, head-and-neck patients may have a different common template. Within patient geometry groups, there may be sub-groups with different common templates. For example, different head-and-neck patients may have different templates depending on the location of the tumour, or different prostate patients may have different templates depending on the risk level of the patient.

In step 630, the obtained past utility function or cost function is used to obtain a baseline dose distribution that was used for similar past patients. For example, an optimisation process may be used on the past utility function or past cost function to determine machine parameters that minimise utility or maximise cost. These machine parameters would correspond to a dose distribution that may be used as the baseline dose distribution. The past utility function or past cost function may be based on past clinical goals.

As an optional further step, a human user may make some modifications to the dose distribution obtained using the past utility function or past cost function. For example, an expert highly experienced treatment planner may notice that a certain metric associated with the dose distribution obtained using the past utility function or past cost function is not acceptable, and may make amendments to the dose distribution accordingly.

As noted above, the baseline dose distribution does not have to be realisable by a radiation therapy treatment system. Thus, the optimisation process performed using the past utility function or the past cost function is not constrained by achievable machine parameters. For example, the optimisation may be performed entirely in "fluence space", that is, the optimisation aims to optimise the dose distribution, and not the machine parameters such as multileaf collimator (MLC) leaf positions or gantry angles. This would lead to a baseline dose distribution that cannot be delivered exactly by the treatment machine (for example, the MLC leaf apertures may provide a dose that is not conformal to the tumour), or the treatment plan may be impractical (for example, excessive required MLC leaf movements would result in an excessively long treatment time).

In step 640, the dose distribution obtained from the past utility function or the past cost function is used as a baseline dose distribution. For example, a baseline dose distribution obtained in step 640 may be used in the method as illustrated in FIG. 3 and as described above in relation to FIG. 3; that is, the baseline dose distribution obtained in step 640 may be used as a starting point in a radiation therapy treatment plan optimisation process.

*Past similar patients*

As discussed above, dose prediction models, past utility functions or past cost functions that were used for similar past patients are used to determine the baseline dose distribution. Similar patients may be grouped in multiple ways.

Some examples include:

- Treatment site (e.g. Intra-cranial, Head-and-Neck, Lung, Breast, Liver, Prostate, etc)
- Laterality (e.g., uni-lateral Head-and-Neck, bi-lateral Head-and-Neck)
- Risk level (e.g. low-risk Prostate, high-risk Prostate)
- Fractionation scheme (e.g. Sequential Head-and-Neck, Simultaneously integrated boost Head-and-Neck, SBRT-lung, normal Lung)

Examples of clinical goals and cost functions will now be discussed.

Clinical goals

The utility function or cost function for a new patient may be calibrated using clinical goals (as well as e.g. a baseline distribution, significant deviations from clinical goals, etc.).

In some embodiments, the clinical goals may specify that in the eventual treatment plan, a particular plan metric should be greater or smaller than a given threshold value. In one example, a user may have some initial clinical goal template for a patient, where the template is a prioritised list of clinical goals. Such a template may be based on clinical goals used for past similar patients.

Some examples of clinical goals or plan metrics include the following:

*Volume-at-dose:* For a specified structure (organ or target), for the specified dose level, what is the volume (either absolute in cubic-cm or relative as percentage of the total volume of the associated organ) of the structure where the dose is equal to or greater than the specified dose level. Volume-at-dose may refer to an amount of structure volume that receives at least the amount of dose given (i.e. the volume axis value in the structure's DVH curve at a given dose level).

*Dose-at-Volume:* For a specified structure, for a specified volume, what is the dose level, where the corresponding Volume-at-Dose metric (with that dose level) would be equal to the specified volume. Dose-at-volume is the dose axis value of the structure's DVH curve at a certain volume level.

*Max Dose:* a dose-at-volume when the volume is zero.

*Min Dose:* a dose-at-volume when the volume is 100%.

*Mean dose:* mean dose in the specified structure.

*Geometrically equivalent uniform dose (GEUD):* a generalisation of the mean dose, where each dose voxel value is first raised to a specified power before the average is calculated (and the specified power is other than 0 or 1).

*Max dose at distance:* the maximum dose within a certain distance from the structure surface.

*Conformality:* the size of the volume surrounding target structure (including the target itself) where the dose is at least 50% of the prescription dose level.

*Homogeneity:* the deviation of voxel doses inside a target structure.

There can be multiple different thresholds associated to each goal. For example, a 'per protocol' threshold may describe a more strict threshold the planner should aim for. An 'acceptable variation' (or 'minor variation') may be a threshold describing what would still be acceptable in case one cannot reach the 'per protocol' value. These two different values can be considered as two separate goals referring to the same metric (but having possibly different priority). An "unacceptable variation" (or "major variation") may be a threshold beyond which a metric would be considered intolerable.

*Practical examples of clinical goals*

For purposes of illustration, an exemplary set of clinical goals for use in head & neck radiation therapy is as follows:

Table 2: Typical set of prioritised clinical goals

|  | **Per Protocol** | **Minor Variation** | **Major Variation** |
|---|---|---|---|
| Total dose to PTV1 (e.g. to 95% of PTV1) | 60-64 Gy | 58-60 or 64-66 Gy | <58 or >66 Gy |
| Minimum dose ("cold spot") within PTV1 | 56-60 Gy | 54-56 Gy | < 54Gy |
| Maximum dose ("hot spot") within PTV1 | < 70 Gy | 70-72 Gy | > 72Gy |
| Maximum dose ("hot spot") outside of PTV1 | < 66 Gy | 66-70 Gy | > 70Gy |
| Total dose to PTV2 (e.g. to 95% of PTV2) | 56-60 | 54-56 or 60-62 | <54 or >62 |
| Minimum dose ("cold spot") within PTV2 | 52-56 | 50-52 | <50 |
| Total dose to PTV3 (e.g. to 95% of PTV3) | 66-70 | 64-66 or 70-72 | <64 or >72 |
| Minimum dose ("cold spot") to PTV3 | 62-66 | 60-62 | <60 |
| Total dose to Spinal Cord (first volume) | <48 Gy | 48-50 Gy | > 50 Gy |
| Total dose to Spinal Cord (second volume < first volume) | < 50 Gy | 50-52 Gy | > 52 Gy |

In the above table, 'Major Variant' refers to a major deviation from the protocol. The difference between the per protocol value and the major variant provides the practical upper limit for defining the significance of the change, although a smaller value may also be chosen for the significant change (such as half or one third of the difference between the per protocol value and the major variant). An insignificant change may be defined, for example, to be an order of magnitude smaller than this difference. The column 'minor variants' describes the range when the plan can still be considered as essentially following the protocol.

Exemplary cost functions

*Example cost function #1*

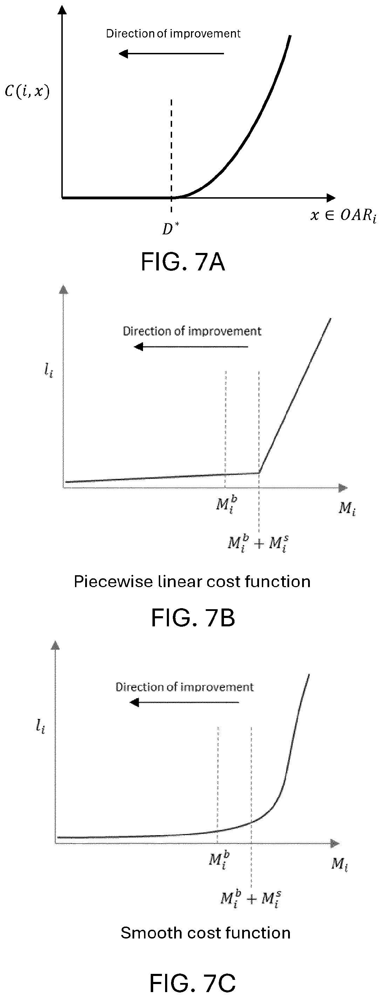

Cost functions may comprise a weighted sum of quadratic objectives (as illustrated in FIG. 7A), of the following form:

$$C = \sum_i w_i \sum_{x \in OAR_i} \max(0, D(x) - D^*)^2$$

Equation 1

where:

C is the cost function
x is a voxel in the dose matrix
$D(x)$ is a dose in that voxel
D* is a target dose level defined for example using a DVH point objective (D* is the dose level of the objective), Volume-at-Dose (D* is the goal dose value), or Dose-at-volume goal (D* is the dose associated to the goal volume)
$OAR_i$ is the organ associated to objective i
$w_i$ is the weight associated to the objective i
i is the index of the objective

Note that Equation 1 is a double summation over all objectives *i* and over all voxels *x.* The example of FIG. 7A shows only the contribution of a single voxel *x* to the cost function C.

*Example cost function #2*

With comparison to Example #1, a more generic way of defining the cost function comprises:

$$C = \sum_i w_i (\max(0, M_i - M_i^*))^2$$

<div align="right">Equation 2</div>

where:

C is the cost function
$M_i$ is the value of the metric i (DVH point objective value, dose-to-volume, volume-to-dose, mean-dose, $D_{max}$, GEUD etc)
$M_i^*$ is the goal value associated to the metric i
$w_i$ is the weight
i is the index of the objective

This exemplary cost function assumes that the goals are of the type wherein the desired value of the metrics $M_i^*$ is smaller than the goal value, which is consistent with the use of the "max" function. In an alternative example, the desired value of the metrics $M_i^*$ is greater than the goal value, in which case the "min" function would be used instead of the "max" function).

*Example cost function #3*

Cost functions according to some embodiments may comprise objectives that are based on a baseline dose distribution. Further, the relative significance or insignificance of goal improvements and/or goal violations are taken into account. The present exemplary cost function involves subtracting the baseline value of the metric from the actual value of the metric and normalising this difference by a defined significance value; a sum of the squared normalised differences is then defined as the cost.
An example of such a quadratic cost function is:

$$C = \sum_i \left(\frac{M_i - M_i^b}{M_i^s}\right)^2$$

<div align="right">Equation 3</div>

where:

C is the cost function
$M_i$ is the value of the metric i (DVH point objective value, dose-to-volume, volume-to-dose, mean-dose, $D_{max}$, GEUD, etc)
$M_i^b$ is the metric value in the baseline
$M_i^s$ is the significance of the change of the metric i around the base-line value
i is the index of the objective

The purpose of the factor $M_i^s$ is to make different terms in the sum associated with different metrics to have comparable numerical values. As an example, a scenario using only two numerical goals may have the following cost function:

$$C = \left(\frac{M_1 - M_1^b}{M_1^s}\right)^2 + \left(\frac{M_2 - M_2^b}{M_2^s}\right)^2$$

In this example, we assume:

Metric 1 $M_1$ has a base line value $M_1{}^b$ of 30Gy
Metric 1 $M_1$ has a significant deviation defined to be 5Gy
Metric 2 $M_2$ has a base line value of 50% (e.g. of organ volume)
Metric 2 $M_2$ has a significant deviation defined to be 2%

The cost function may be evaluated to determine which of multiple plan candidates is the best. As an example, we consider two treatment plans, referred to as "plan candidate A" and "plan candidate B". We assume that plan candidate A has $M_1$ = 35Gy and $M_2$ = 52%. The cost function for plan candidate A is 2. We assume plan candidate B has $M_1$ = 32.5 Gy and $M_2$ = 60%. The cost function for plan candidate B is 25.25. So, based on the cost function, plan candidate A is better than plan candidate B reflecting the fact that plan A has a significant improvement (relative to plan B) for the $M_2$ metric but only an insignificant degradation (relative to plan B) for the $M_1$ metric.

*Example cost function #3 - variant #1*

A variation of Equation 3 comprises:

$$ C = \sum_i \left( \max\left(0, \frac{M_i - M_i^b}{M_i^s}\right) \right)^2 $$

Equation 3A

Equation 3A expresses that it is generally beneficial to reduce the metric values and thus any metric value smaller than the base line is considered to not be worse than the base line.

*Example cost function #3 - variant #2*

A further variation of Equation 3 comprises:

$$ C = \sum_i \frac{M_i - M_i^b}{M_i^s} $$

Equation 3B

This is the same as Equation 3, except that instead of a sum of squared normalised differences from the baseline, Equation 3B is simpler and is a sum of normalised differences from the baseline (the normalisation is with respect to significance).

*Example cost function #4*

In some embodiments, the cost function comprises a piecewise linear (PWL) cost function that uses a baseline dose distribution as well as significance:

$$ C = \sum_i l_i(M_i) $$

Equation 4

where:

C is the cost function
$l_i$ is a piecewise-linear function of a shape as shown in FIG. 7B
$M_i$ is the value of the metric i

In the graph of FIG. 7B, at the vicinity of the desired goal metric, piecewise linear (PWL) function segments are nearly horizontal, reflecting that any small changes in the vicinity of the goal metric are not significant. At a greater distance from the desired goal metric, the PWL function segments may have a steeper gradient, reflecting than any larger changes away from the goal metric are significant (and possibly unacceptable) changes.

As an alternative to Example #4, some embodiments may use "smoother" cost functions than a piecewise linear function. An example is shown in FIG. 7C.

*Utility functions*

The above four examples relate to cost functions. Exemplary utility functions that can be used with the present method would be similar to the above described cost functions, except that the direction of improvement (as indicated in FIGS. 7A-7C) would be in the opposite direction. That is, while lower values of a cost function are more desirable and higher values of a cost function are less desirable, lower values of a utility function are less desirable and higher values of a utility function are more desirable.

Computer system

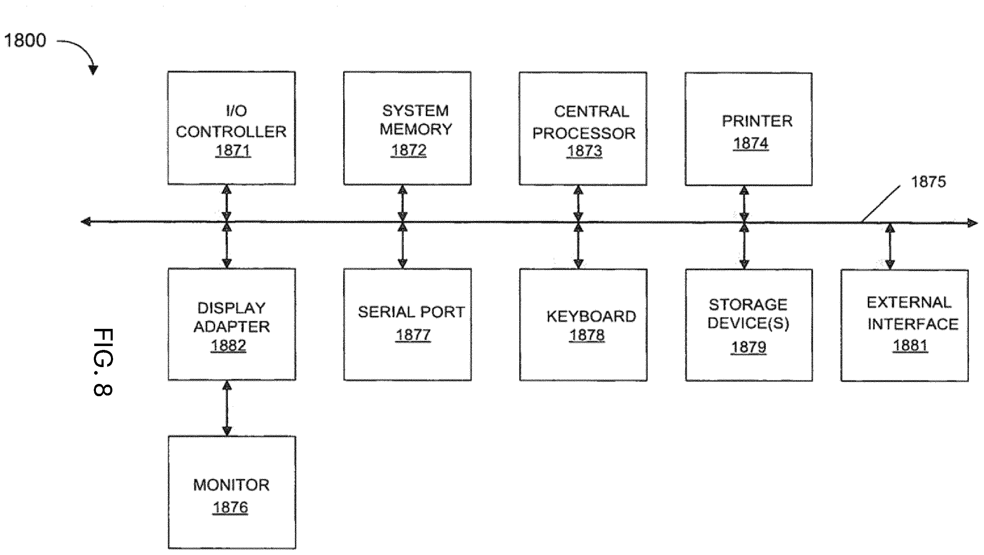

Any of the methods mentioned herein may utilise a computer system or any suitable number of computer subsystems. Examples of such subsystems are shown in FIG. 8 in computer system 1800. In some embodiments, a computer system includes a single computer apparatus, where the subsystems can be the components of the computer apparatus. In other embodiments, a computer system can include multiple computer apparatuses, each being a subsystem, with internal components.

The subsystems shown in FIG. 8 are interconnected via a system bus 1875. Additional subsystems such as a printer 1874, keyboard 1878, storage device(s) 1879, monitor 1876, which is coupled to display adapter 1882, and others are shown. Peripherals and input/output (I/O) devices, which couple to I/O controller 1871, can be connected to the computer system by any number of means known in the art, such as serial port 1877. For example, serial port 1877 or external interface 1881 (e.g. Ethernet, Wi-Fi, etc.) can be used to connect computer system 1800 to a wide area network such as the Internet, a mouse input device, or a scanner. The interconnection via system bus 1875 allows the central processor 1873 to communicate with each subsystem and to control the execution of instructions from system memory 1872 or the storage device(s) 1879 (e.g., a fixed disk, such as a hard drive or optical disk), as well as the exchange of information between subsystems. The system memory 1872 and/or the storage device(s) 1879 may embody a computer readable medium. Any of the data mentioned herein can be output from one component to another component and can be output to the user.

A computer system can include a plurality of the same components or subsystems, e.g., connected together by external interface 1881 or by an internal interface. In some embodiments, computer systems, subsystems, or apparatuses can communicate over a network. In such instances, one computer can be considered a client and another computer a server, where each can be part of a same computer system. A client and a server can each include multiple systems, subsystems, or components.

It should be understood that any of the embodiments of the present invention can be implemented in the form of control logic using hardware (e.g. an application specific integrated circuit or field programmable gate array) and/or using computer software with a generally programmable processor in a modular or integrated manner. As used herein, a processor includes a multi-core processor on a same integrated chip, or multiple processing units on a single circuit board. Based on the disclosure and teachings provided herein, a person of ordinary skill in the art will know and appreciate other ways and/or methods to implement embodiments of the present invention using hardware and a combination of hardware and software.

Any of the computer implemented methods described in this application may be implemented as software code to be executed by a processor using any suitable computer language such as, for example, Java, C++ or Perl using, for example, conventional or object-oriented techniques. The software code may be stored as a series of instructions or commands on a computer readable medium for storage and/or transmission; suitable media include random access memory (RAM), a read only memory (ROM), a magnetic medium such as a hard-drive or a floppy disk, or an optical medium such as a compact disk (CD) or DVD (digital versatile disk), flash memory, and the like. The computer readable medium may be any combination of such storage or transmission devices.

Such programs may also be encoded and transmitted using carrier signals adapted for transmission via wired, optical, and/or wireless networks conforming to a variety of protocols, including the Internet. As such, a computer readable medium according to an embodiment of the present invention may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps of the methods. Thus, embodiments can be directed to computer systems configured to perform the steps of any of the methods described herein, potentially with different components performing a respective step or a respective group of steps. Although presented as numbered steps, steps of methods herein can be performed at a same time or in a different order. Additionally, portions of these steps may be used with portions of other steps from other methods. Also, all or portions of a step may be optional. Additionally, any of the steps of any of the methods can be performed with modules, circuits, or other means for performing these steps.

Those skilled in the art will recognise that a wide variety of modifications, alterations, and combinations can be made with respect to the above-described embodiments without departing from the scope of the invention, and that such modifications, alterations, and combinations are to be viewed as being within the ambit of the inventive concept.

**Claims**

1. A method for computer-assisted radiation therapy treatment planning, comprising:

   receiving a desired baseline dose distribution based on dose distributions used in radiation therapy treatment planning for one or more past patients that are similar to a new patient;
   receiving or defining a set of clinical goals;
   using the baseline dose distribution to calibrate a utility function or cost function based on the set of clinical goals; and
   using the utility function or cost function to optimise a treatment plan.

2. The method of claim 1, wherein the received desired baseline dose distribution comprises a dose distribution used for a past patient similar to the new patient.

3. The method of claim 1 or claim 2, further comprising:

   selecting a dose prediction model constructed for past patients similar to the new patient, from a library of dose prediction models;
   using the selected dose prediction model to generate the desired baseline dose distribution.

4. The method of any preceding claim, further comprising:

   selecting a template utility function or a template cost function constructed for past patients similar to the new patient, from a library of template utility functions or template cost functions; and
   using the selected template utility function or template cost function to generate the desired baseline dose distribution.

5. The method of any preceding claim, wherein the received desired baseline dose distribution is partially or wholly defined by a human user.

6. The method of any preceding claim, wherein the received desired baseline dose distribution is further modified by a human user.

7. The method of any of claim 1 to claim 4, wherein the received desired baseline dose distribution is partially or wholly defined by a computer-implemented method.

8. The method of any preceding claim, wherein the baseline dose distribution is generated based on relaxed machine parameter constraints.

9. The method of any preceding claim, further comprising receiving information defining one or more of:

   a relative priority of each clinical goal;
   significant deviations from clinical goals, and/or
   insignificant deviations from clinical goals; and
   using the baseline dose distribution and the received information defining the relative priority of each clinical goal, significant deviations from clinical goals, and/or insignificant deviations from clinical goals to calibrate the utility function or cost function.

10. The method of claim 9, wherein the information defining clinical goal priorities, significant deviations from clinical goals and/or insignificant deviations from clinical goals is received from a library of templates defining clinical goal priorities, significant deviations from clinical goals and/or insignificant deviations from clinical goals in past similar patients.

11. The method of claim 9 or claim 10, wherein the information defining clinical goal priorities, significant deviations from clinical goals and/or insignificant deviations from clinical goals is at least partially received from a human user.

12. The method of any preceding claim, wherein the cost function comprises a sum of quadratic terms, each quadratic term penalising a deviation from a corresponding clinical goal, and optionally wherein each quadratic term is normalised by significance;
and/or:
wherein the utility function or cost function comprises a piecewise linear function.

13. The method of any preceding claim, wherein the utility function or cost function is assessed by determining a rating that depends on the extent to which the desired baseline dose distribution is achieved;
and optionally wherein:

> the rating is a maximal value when all clinical goals are achieved;
> the rating is a minimal value when one or more of achieved metrics deviate from corresponding clinical goals by more than a significant amount;
> the rating is between the maximal value and the minimal value in all other cases.

14. A radiation treatment planning computer system comprising a memory and a processor configured to perform the method as defined in any one of claims 1-13.

15. A computer program product comprising a non-transitory, computer readable storage medium encoded with instructions operable for execution by a processor to perform the method as defined in any one of claims 1-13.

FIG. 1

FIG. 2

300

| 310 | Receive desired baseline dose distribution |
|---|---|

↓

| 320 | Receive / define a prioritised list of clinical goals |
|---|---|

↓

| 330 | Define significant / insignificant deviations from clinical goals |
|---|---|

↓

| 340 | Calibrate cost or utility function using baseline dose distribution, clinical goals, clinical goal priorities, and information regarding significant / insignificant deviations from clinical goals |
|---|---|

↓

| 350 | Optimise planned dose distribution and planned machine parameters by minimising cost function / maximising utility function |
|---|---|

↓

| 360 | Obtain treatment plan |
|---|---|

FIG. 3

FIG. 4

✓ = change accepted
✗ = change rejected

| | | | LOW PRIORITY GOAL | | | |
|---|---|---|---|---|---|---|
| | | | Improvement | | Worsening | |
| | | | Significant | Insignificant | Significant | Insignificant |
| HIGH PRIORITY GOAL | Improvement | Significant | ✓ 401 | ✓ 401 | ✓ 421 | ✓ 422 |
| | | Insignificant | ✓ 401 | ✓ 401 | ✗ | ✓ 423 |
| | Worsening | Significant | ✗ 431 | ✗ 432 | ✗ 411 | ✗ 411 |
| | | Insignificant | ✓ | ✗ 433 | ✗ 411 | ✗ 411 |

470

480

500

```
┌─────────────────────────────┐
│   Receive new patient data  │──── 510
└─────────────────────────────┘
              │
              ▼
┌─────────────────────────────┐
│  Obtain dose prediction model│
│ developed for similar past   │──── 520
│  patients from knowledge base│
└─────────────────────────────┘
              │
              ▼
┌─────────────────────────────┐
│ Use dose prediction model to │
│  obtain dose distribution    │──── 530
│  used for similar past       │
│        patients              │
└─────────────────────────────┘
              │
              ▼
┌─────────────────────────────┐
│ Use dose distribution used   │
│ for similar past patients as │──── 540
│  desired baseline dose       │
│       distribution           │
└─────────────────────────────┘
```

FIG. 5

600

```
Receive new patient data                                    610
            |
            v
Obtain utility (or cost) function
developed for similar past patients from                    620
library of utility (or cost) function
templates
            |
            v
Use obtained utility (or cost)
function to obtain dose distribution                         630
used for similar past patients
            |
            v
Use dose distribution used for
similar past patients as baseline                           640
dose distribution
```

FIG. 6

Direction of improvement

$C(i, x)$

$D^*$

$x \in OAR_i$

FIG. 7A

Direction of improvement

$l_i$

$M_i^b$

$M_i^b + M_i^s$

$M_i$

Piecewise linear cost function

FIG. 7B

Direction of improvement

$l_i$

$M_i^b$

$M_i^b + M_i^s$

$M_i$

Smooth cost function

FIG. 7C

FIG. 8

Europäisches
Patentamt

European
Patent Office

Office européen
des brevets

# EUROPEAN SEARCH REPORT

Application Number

EP 24 21 2019

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/226603 A1 (PELTOLA JARKKO [FI] ET AL) 11 July 2024 (2024-07-11)<br>* figures 4, 6, 7 *<br>* paragraph [0060] *<br>* paragraphs [0074] - [0081] *<br>* paragraphs [0090] - [0091] *<br>* paragraphs [0099] - [0114] *<br>* paragraphs [0260] - [0263] *<br>----- | 1-15 | INV.<br>A61N5/10 |
| X | US 2017/259082 A1 (BZDUSEK KARL ANTONIN [US] ET AL) 14 September 2017 (2017-09-14)<br>* figure 2 *<br>* paragraphs [0015] - [0016] *<br>* paragraphs [0030] - [0041] *<br>* paragraphs [0045] - [0047] *<br>----- | 1-15 | |
| X<br>A | US 2015/273238 A1 (KUMAR PRASHANT [IN] ET AL) 1 October 2015 (2015-10-01)<br>* paragraphs [0035] - [0042] *<br>* figure 2 *<br>* claims 1, 6 *<br>----- | 1,5-15<br>2-4 | |

TECHNICAL FIELDS
SEARCHED (IPC)

A61N

The present search report has been drawn up for all claims

| Place of search | Date of completion of the search | Examiner |
|---|---|---|
| Munich | 21 March 2025 | Seiferle, Benedict |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 

& : member of the same patent family, corresponding
    document

EPO FORM 1503 03.82 (P04C01)

**EP 4 741 004 A1**

## ANNEX TO THE EUROPEAN SEARCH REPORT
## ON EUROPEAN PATENT APPLICATION NO.

EP 24 21 2019

21-03-2025

| Patent document cited in search report | Publication date | Patent family member(s) | | Publication date |
|---|---|---|---|---|
| US 2024226603 A1 | 11-07-2024 | CN | 114364435 A | 15-04-2022 |
| | | EP | 4028121 A1 | 20-07-2022 |
| | | US | 2021069527 A1 | 11-03-2021 |
| | | US | 2022161058 A1 | 26-05-2022 |
| | | US | 2024226603 A1 | 11-07-2024 |
| | | WO | 2021048143 A1 | 18-03-2021 |
| US 2017259082 A1 | 14-09-2017 | CN | 107278303 A | 20-10-2017 |
| | | EP | 3227809 A1 | 11-10-2017 |
| | | JP | 6845136 B2 | 17-03-2021 |
| | | JP | 2017536190 A | 07-12-2017 |
| | | US | 2017259082 A1 | 14-09-2017 |
| | | WO | 2016088075 A1 | 09-06-2016 |
| US 2015273238 A1 | 01-10-2015 | CN | 105120949 A | 02-12-2015 |
| | | EP | 2912585 A2 | 02-09-2015 |
| | | JP | 6317749 B2 | 25-04-2018 |
| | | JP | 2015532869 A | 16-11-2015 |
| | | US | 2015141733 A1 | 21-05-2015 |
| | | US | 2015273238 A1 | 01-10-2015 |
| | | WO | 2014068435 A2 | 08-05-2014 |

EPO FORM P0459